(19) Europäisches Patentamt
European Patent Office
Office européen des brevets

(11) **EP 1 232 478 B1**

(12) **EUROPÄISCHE PATENTSCHRIFT**

(45) Veröffentlichungstag und Bekanntmachung des Hinweises auf die Patenterteilung:
**13.07.2011 Patentblatt 2011/28**

(21) Anmeldenummer: **00962377.8**

(22) Anmeldetag: **24.08.2000**

(51) Int Cl.:
***G06N 3/08*** (2006.01)

(86) Internationale Anmeldenummer:
**PCT/EP2000/008280**

(87) Internationale Veröffentlichungsnummer:
**WO 2001/015078 (01.03.2001 Gazette 2001/09)**

(54) **VERFAHREN ZUM TRAINIEREN EINES NEURONALEN NETZES**

METHOD FOR TRAINING A NEURAL NETWORK

PROCEDE DESTINE A L'APPRENTISSAGE D'UN RESEAU NEURONAL

(84) Benannte Vertragsstaaten:
**AT BE CH CY DE DK ES FI FR GB GR IE IT LI LU MC NL PT SE**

(30) Priorität: **26.08.1999 DE 19940577**

(43) Veröffentlichungstag der Anmeldung:
**21.08.2002 Patentblatt 2002/34**

(73) Patentinhaber:
• **Harbeck, Nadia, Dr.**
  **83624 Otterfing (DE)**
• **Kates, Ronald Dr.**
  **83624 Otterfing (DE)**

(72) Erfinder:
• **KATES, Ronald**
  **83624 Otterfing (DE)**
• **HARBECK, Nadia**
  **83624 Otterfing (DE)**

• **SCHMITT, Manfred**
  **81669 München (DE)**

(74) Vertreter: **Grünecker, Kinkeldey, Stockmair & Schwanhäusser Anwaltssozietät Leopoldstrasse 4 80802 München (DE)**

(56) Entgegenhaltungen:
**US-A- 5 528 700    US-A- 5 687 286
US-A- 5 734 797    US-A- 5 812 992**

• **LE CUN Y.; DENKER J.; SOLLA S.: 'Optimal Brain Damage' ADVANCES IN NEURAL INFORMATION PROCESSING SYSTEMS Bd. 2, 1990, SAN MATEO, XP002256372**
• **NELDER J.A.; MEAD R.: 'A simplex method for function minimization' COMPUTER JOURNAL Bd. 7, Nr. 4, Januar 1965, Seiten 308 - 313, XP000574342**

**Beschreibung**

## 1. FACHGEBIET DER ERFINDUNG

Die Erfindung betrifft ein Verfahren zum Trainieren eines neuronalen Netzes zur Ermittelung von Risikofunktionen für Patienten im Anschluß an eine Ersterkrankung mit einer vorbestimmten Krankheit auf Grundlage vorgegebener Trainings-Datensätze, welche objektivierbare und zu einem erheblichen Teil meßtechnisch erfaßte Daten zum Krankheitsbild der Patienten beinhalten, wobei das neuronale Netz eine Eingangsschicht mit einer Mehrzahl von Eingangs-Neuronen und wenigstens eine Zwischenschicht mit einer Mehrzahl von Zwischen-Neuronen umfaßt, sowie ferner eine Ausgangsschicht mit einer Mehrzahl von Ausgangs-Neuronen und eine Vielzahl von Synapsen, die jeweils zwei Neuronen unterschiedlicher Schichten miteinander verbinden.

## 2. FACHLICHER HINTERGRUND - STAND DER TECHNIK

### 2.1. Allgemeines

Auf zahlreichen Gebieten sind neuronale Netze zur Auswertung großer Datenmengen zu den bislang üblichen Auswerteverfahren ergänzend hinzugetreten oder haben diese ersetzt. Es hat sich nämlich gezeigt, daß neuronale Netze besser als die herkömmlichen Verfahren in der Lage sind, in den Datenmengen verborgene, nicht ohne weiteres erkennbare Abhängigkeiten zwischen den einzelnen Eingangsdaten aufzuspüren und aufzuzeigen. Daher liefern neuronale Netze, welche anhand einer bekannten Datenmenge trainiert worden sind, für neue Eingangsdaten des gleichen Datentyps zuverlässigere Aussagen als die bisherigen Auswerteverfahren.

Die US -A- 5 734 797 offenbart ein System zur Generierung eines minmalen künstlichen neuronalen Netzwerks, das als Eingänge die minimale Anzahl an Merkmalen aufweist, die dazu notwendig ist, zwischen verschiedenen Ereignisklassen zu unterscheiden. Ein anfängliches neuronales Netzwerk wird trainiert, und das trainierte neuronale Netzwerk wird anschließend durch Entfernen von Verbindungen und Knoten ausgedünnt.

Im Bereich medizinischer Anwendungen ist es beispielsweise bekannt, neuronale Netze zur Bestimmung einer Überlebensfunktion für an einer bestimmten Krankheit, etwa Krebs, leidende Patienten einzusetzen. Diese Überlebensfunktion gibt dabei in Abhängigkeit der seit der Ersterkrankung vergangenen Zeit die Wahrscheinlichkeit an, daß bei einem betrachteten Patienten ein vorbestimmtes Ereignis eintritt. Dieses vorbestimmte Ereignis muß dabei nicht notwendigerweise der Tod des Patienten sein, wie dies die Bezeichnung "Überlebensfunktion" vermuten läßt, sondern kann ein beliebiges Ereignis sein, beispielsweise eine erneute Erkrankung an Krebs.

Die Datensätze umfassen eine ganze Reihe von objektivierbaren Angaben, d.h. Daten, auf deren Wert eine das neuronale Netz gegebenenfalls bedienende Person keinen Einfluß hat und deren Wert gewünschtenfalls automatisch erfaßt werden kann. Im Falle von Brustkrebs sind dies Angaben über die Person des Patienten, beispielsweise Alter, Geschlecht und dergleichen, Angaben über das Krankheitsbild, beispielsweise Anzahl der von Krebs befallenen Lymph-Knoten, tumor-biologische Faktoren, wie upA (Urokinase-Plasminogen-Aktivator), sein Inhibitor PAI-1 und dergleichen Faktoren, sowie Angaben über die Behandlungsmethode, beispielsweise Art, Dauer und Intensität einer Chemo- oder Strahlen-Therapie. Unnötig zu erwähnen, daß eine ganze Reihe der vorstehend erwähnten Angaben, insbesondere der Angaben zum Krankheitsbild, nur unter Einsatz geeigneter Meßapparaturen ermittelt werden können. Darüber hinaus können auch die personen-bezogenen Daten automatisiert von geeigneten Datenträgern abgelesen werden, beispielsweise maschinenlesbaren Personalausweisen oder dergleichen. Selbstverständlich können die objektivierbaren Daten dann, wenn sie nicht alle gleichzeitig verfügbar sind, was insbesondere bei den Labor-Meßwerten häufig der Fall sein dürfte, auf einem geeigneten Speichermedium in einer Datenbank zwischengespeichert werden, bevor sie dem neuronalen Netz als Eingangsdaten zugeführt werden.

### 2.2. Das neuronale Netz als Signalfilter

Gemäß vorstehendem kann man ein neuronales Netz also als eine Art "Signalfilter" auffassen, das aus einem verrauschten und daher an sich noch nicht aussagekräftigen Eingangssignal ein aussagekräftiges Ausgangssignal herausfiltert. Ob bzw. wie gut das Filter seine Funktion erfüllen kann, hängt wie bei jedem Filter davon ab, ob es gelingt, die Intensität des Eigenrauschens des Filters so niedrig zu halten, daß das herauszufilternde Signal nicht in diesem Eigenrauschen untergeht.

Die Intensität des "Eigenrauschens" eines neuronalen Netzes ist umso niedriger, je mehr Datensätze einerseits zum Trainieren des neuronalen Netzes zur Verfügung stehen und je einfacher andererseits die Struktur des neuronalen Netzes ist. Zudem steigt die Generalisierungsfähigkeit des Netzes mit zunehmender Vereinfachung der Struktur des neuronalen Netzes an. Bei einer im Stand der Technik üblichen Vorgehensweise befaßt sich daher ein Teil des Trainings neuronaler Netze mit dem Auffinden und Eliminieren von Strukturteilen, die für den Erhalt eines aussagekräftigen Ausgangssignals entbehrlich sind. Bei diesem "Ausdünnen" (in der Fachsprache auch als "pruning" bezeichnet) ist allerdings als weitere Randbedingung zu beachten, daß die Struktur des neuronalen Netzes nicht beliebig "ausgedünnt" werden darf, da mit abnehmender Komplexität des neuronalen Netzes auch dessen Fähigkeit, komplexe Zusammenhänge nachzubilden, und somit dessen Aussagekraft abnimmt.

### 2.3. Probleme beim medizinischen Einsatz

Nun steht man in der Praxis, insbesondere bei dem eingangs angesprochenen medizinischen Einsatz neuronaler Netze, häufig vor dem Problem, daß lediglich sehr kleine Datenmengen von typischerweise wenigen hundert Datensätzen für das Training des neuronalen Netzes zur Verfügung stehen. Erschwerend kommt hinzu, daß für das Training nicht nur eine TrainingsDatenmenge, sondern ferner auch eine Validierungs-Datenmenge und eine Generalisierungs-Datenmenge vorgesehen werden müssen. Auf die Bedeutung dieser beiden Datenmengen wird weiter unten unter den Punkten 5.5. bzw. 5.7. noch näher eingegangen werden.

Bei derart geringen Datenmengen führte der Einsatz bekannter Pruning-Methoden aber stets zu einer so starken Vereinfachung der Struktur des neuronalen Netzes, daß die Aussagekraft des neuronalen Netzes auf ein nicht zu tolerierendes Maß absank. Um nach Abschluß der Trainungsphase aber dennoch neuronale Netze erhalten zu können, welche aussagekräftige Ausgangssignale liefern, wurden daher im Stand der Technik bei Vorliegen lediglich kleiner Trainingsdatenmengen neuronale Netze mit einer starren, d.h. fest vorgegebenen und unveränderlichen Struktur eingesetzt. Dabei wurde der Komplexitätsgrad bzw. die Einfachheit dieser starren Struktur auf Grundlage von Erfahrungswissen derart gewählt, daß das neuronale Netz einerseits eine hohe Aussagekraft und andererseits ein gerade noch tolerierbares Eigenrauschen aufwies. Bislang wurde davon ausgegangen, daß die Vorgabe einer unveränderlichen Struktur unumgänglich sei.

Ein weiteres Problem der medizinischen Anwendungen neuronaler Netze ist darin zu sehen, daß zum Training nur "zensierte" Daten vorliegen. Mit dem Begriff "Zensur" wird dabei der Umstand umschrieben, daß für Patienten, die zum Zeitpunkt der Datenerfassung glücklicherweise noch keinen Rückfall erlitten haben, die zukünftige Entwicklung nicht vorauszusehen ist, und Aussagen über die Überlebensfunktion daher nur bis zum Zeitpunkt der Erhebung der Daten möglich sind.

Es braucht nicht betont zu werden, daß gerade bei medizinischen Anwendungen auf ein wirklich aussagekräftiges Ergebnisses unter gar keinen Umständen verzichtet werden darf. Es ist nämlich in keinem Fall akzeptabel, wenn auch nur einem Patient eine Behandlung nur deshalb vorenthalten würde, weil das neuronale Netz diese nicht für notwendig erachtet hat. Die Folgen für den Patienten könnten unabsehbar sein.

Hinsichtlich der Einzelheiten des vorstehend geschilderten Standes der Technik sei auf die unter Punkt 6. "Literaturverzeichnis" aufgeführten Aufsätze verwiesen.

### 3. AUFGABE DER ERFINDUNG

Demgegenüber ist es Aufgabe der Erfindung, ein automatisches Verfahren zum Trainieren eines neuronalen Netzes zur Ermittelung von Risikofunktionen für Patienten im Anschluß an eine Ersterkrankung mit einer vorbestimmten Krankheit bereitzustellen, welches es trotz einer geringen Anzahl von zur Verfügung stehenden Trainingsdatensätzen erlaubt, ein neuronales Netz variabler Struktur einzusetzen und dessen Struktur in wenigstens einem Strukturvereinfachungs-Schritt zu optimieren.

### 4. LÖSUNG DER AUFGABE

Diese Aufgabe wird erfindungsgemäß gelöst durch ein Verfahren zum Trainieren eines neuronalen Netzes gemäß Anspruch 1.

Ein in der im Anspruch 1 beschriebenen Art und Weise trainiertes neuronales Netz unterstützt den behandelnden Arzt beispielsweise bei der Entscheidung, welche Nachbehandlung er bei einem bestimmten frisch operierten Patienten einsetzen soll. Der Arzt kann hierzu dem neuronalen Netz die Patientendaten und die im Labor meßtechnisch erfaßten Daten über das Krankheitsbild der Erstbehandlung eingeben und erhält von dem neuronalen Netz eine Aussage darüber, bei welcher Nachbehandlungsart sich die für den gerade betrachteten Patienten günstigste Überlebensfunktion ergibt. Selbstverständlich kann auch die Aggressivität der einzelnen Nachbehandlungsarten berücksichtigt werden, so daß bei gleich oder annähernd gleich günstiger Überlebensfunktion die für den Patienten schonendste Art der Nachbehandlung ausgewählt werden kann.

### 5. AUSFÜHRUNGSBEISPIEL

Die Erfindung wird im folgenden an einem Ausführungsbeispiel näher erläutert werden.

## 5.1. Aufbau neuronaler Netze

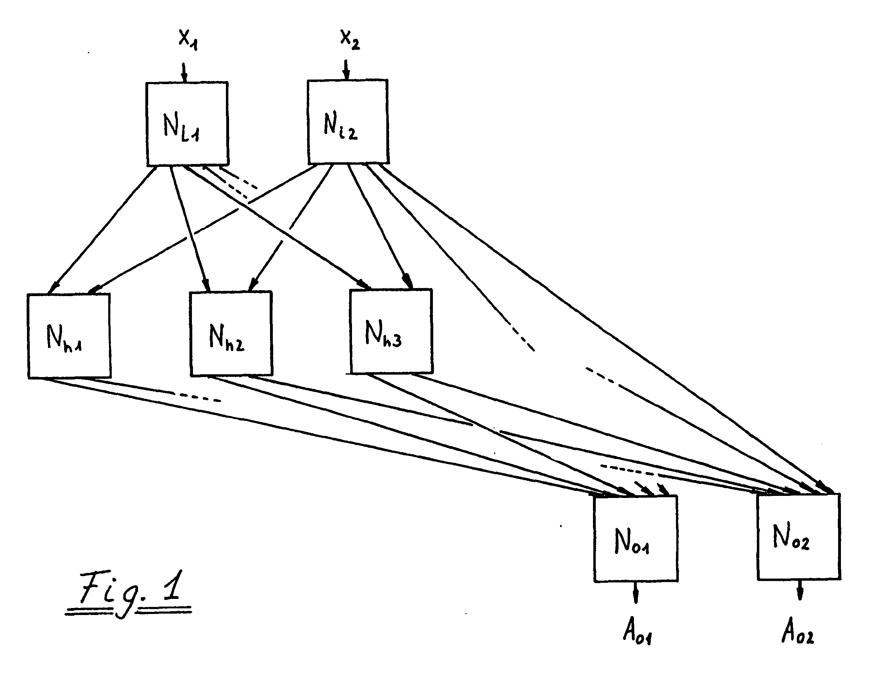

Fig. 1 zeigt den Aufbau eines neuronalen Netzes, das nach Art eines Vielschicht-Perzeptrons aufgebaut ist. In diesem Fall umfaßt das neuronale Netz:

- eine Eingangsschicht mit einer Mehrzahl von Eingangs-Neuronen $N_i$ (i für "input neuron"),
- wenigstens eine Zwischenschicht mit einer Mehrzahl von Zwischen-Neuronen $N_h$ (h für "hidden neuron"),
- eine Ausgangsschicht mit einer Mehrzahl von Ausgangs-Neuronen $N_o$ (o für "output neuron"), und
- eine Vielzahl von Synapsen, die jeweils zwei Neuronen unterschiedlicher Schichten miteinander verbinden.

In der vereinfachten Ausführungsform gemäß Fig. 1, die aus Anschaulichkeitsgründen der nachfolgenden Diskussion zugrundegelegt werden soll, ist lediglich eine einzige Zwischenschicht vorgesehen, und sind die Neuronen (bzw. Knoten, wie sie häufig auch genannt werden) der Ausgangsschicht sowohl mit jedem Neuron der Eingangsschicht als auch mit jedem Neuron der Zwischenschicht über Synapsen (auch als "Konnektoren" bezeichnet) verbunden.

Die Anzahl der Eingangs-Neuronen wird üblicherweise in Abhängigkeit der Anzahl der zur Verfügung stehenden objektivierbaren Angaben gewählt. Sollte hierdurch jedoch die zur Ermittelung der Reaktion des neuronalen Netzes benötigte Zeit in einem nicht zu akzeptierenden Maße ansteigen, so kann beispielsweise mit Hilfe von neuronalen Netzen stark vereinfachter Struktur vorab eine Abschätzung der Bedeutung der einzelnen objektivierbaren Angaben auf die Aussagekraft des Gesamtsystems getroffen werden. Es sei jedoch betont, daß auch diese vorausgehende Abschätzung automatisch und ohne Eingriff der jeweiligen Bedienungsperson erfolgt. Ferner wird die Anzahl der Ausgangs-Neuronen so groß gewählt, daß im Sinne einer Reihenentwicklung der Überlebensfunktion eine ausreichende Anzahl von Reihenentwicklungs-Termen zur Verfügung steht, um eine aussagekräftige Annäherung an die tatsächliche Überlebensfunktion erzielen zu können. Die Anzahl der Zwischen-Neuronen schließlich wird einerseits so groß gewählt, daß die Ergebnisse des trainierten neuronalen Netzes aussagekräftig sind, und andererseits so klein, daß die zur Ermittelung des Ergebnisses benötigte Zeit akzeptabel ist.

## 5.2. Funktion neuronaler Netze

### 5.2.1. Allgemeines

Jedes Neuron empfängt ein Stimulationssignal S, verarbeitet dieses gemäß einer vorbestimmten Aktivierungsfunktion F(S) und gibt ein entsprechendes Antwortsignal A = F(S) aus, das allen unter diesem Neuron angeordneten Neuronen zugeführt wird. Das auf das betrachtete Neuron $N_y$ einwirkende Stimulationssignal $S_y$ wird üblicherweise durch Aufsummieren der Antwortsignale $A_x$ der über diesem Neuron $N_y$ angeordneten Neuronen $N_x$ gebildet, wobei die Beiträge der einzelnen Neuronen $N_x$ jeweils mit einem Gewichtungsfaktor $w_{xy}$ in die Summe eingehen, der die Stärke der die beiden Neuronen verbindenden Synapse angibt.

$$S_y = \sum_x w_{xy} \cdot A_x$$

$$A_y = F(S_y)$$

### 5.2.2. Eingangsschicht

Die Stimulationssignale $S_i$ der Eingangsneuronen $N_i$ werden von den einen bestimmten Patienten j betreffenden Eingabedaten $x_{i,j}$ gebildet.

$$S_i = x_{i,j}$$

Um die Gewichte der Synapsen eines neuronalen Netzes in geeigneter Weise interpretieren zu können, ist es bevorzugt, mit Variablen zu arbeiten, deren Werte in der Größenordnung von 1 liegen. Um dies trotz der üblicherweise recht unterschiedlichen Verteilungen der Eingabedaten erreichen zu können, ist es üblich, die Eingabedaten einer geeigneten Transformation zu unterziehen. Diese Transformation wird von der Aktivierungsfunktion $F_i$ der Eingangs-neuronen vorgenommen:

$$A_i = \tanh[(S_i - S_{i,mittel})/S_{i,Q}]$$

Für die Eingabedaten $x_{i,j}$ wird also zum einen der Mittelwert $S_{i,mittel}$ der der Trainingsdatenmenge angehörenden Patienten j gebildet. Und zum anderen wird ein Skalierungsfaktor $S_{i,Q}$ gebildet. Liegt der Wert einer Eingabevariablen $x_{i,j}$ oberhalb des Mittelwerts $S_{i,mittel}$, so erfolgt die Skalierung gemäß dem 75%-Quartil. Liegt er hingegen unter dem Mittelwert, so erfolgt die Skalierung gemäß dem 25%-Quartil. Durch Einsatz der hyperbolischen Tangens-Funktion als Aktivierungsfunktion $F_i$ schließlich erhält man in einfacher Weise normierte Antwortsignale, deren Werte im Bereich von -1 bis + 1 liegen.

Festzuhalten ist, daß man für Eingangsdaten, die bereits die gewünschte Verteilung, kategorische oder binäre Werte aufweisen, die vorstehende Transformation umgehen kann.

5.2.3. Zwischenschicht

Das Stimulationssignal $S_h$ für die Neuronen $N_h$ der Zwischenschicht wird von der gewichteten Summe der Antwortsignale $A_i$ aller Neuronen $N_i$ der Eingangsschicht gebildet:

$$S_h = \sum_i w_{ih} \cdot A_i$$

Dieses Stimulationssignal $S_h$ wird von den Neuronen $N_h$ gemäß einer vorgegebenen Aktivierungsfunktion $F_h$, die beispielsweise wiederum die hyperbolische Tangens-Funktion sein kann, in ein Antwortsignal $A_h$ umgesetzt:

$$A_h = F_h(S_h - b_h)$$

Die Parameter $b_h$ werden auf dem Fachgebiet der neuronalen Netze als Bias oder "Vorspannung" des betreffenden Neurons bezeichnet. Wie die Werte der Synapsen-Gewichte $w_{xy}$ werden auch die Werte dieser Vorspannungs-parameter $b_h$ im Zuge des Trainings des neuronalen Netzes ermittelt.

5.2.4. Ausgangsschicht

Die Ermittelung des Stimulationssignals $S_o$ und des Antwortsignals $A_o$ für ein Neuron $N_o$ der Ausgangsschicht erfolgt in analoger Weise:

$$S_o = \sum_i w_{io} \cdot (A_i - c_i) + \sum_h w_{ho} \cdot A_h$$

$$A_o = F_o(S_o - b_o)$$

Die Parameter $b_o$ geben wiederum die "Vorspannung" der Neuronen $N_o$ der Ausgangsschicht an, während die Parameter $c_i$ der Anpassung der Stimulationsbeiträge der Neuronen $N_i$ der Eingangsschicht und $N_h$ der Zwischenschicht

dienen. Die Werte sowohl der Parameter $b_o$ als auch der Parameter $c_i$ werden in der Trainingsphase des neuronalen Netzes ermittelt. Hinsichtlich der Vorspannungen $b_o$ kann es dabei vorteilhaft sein, als Randbedingung zu fordern, daß die über die gesamte Trainingsdatenmenge gemittelte Antwort aller Ausgangsneuronen $N_o$ Null ist. Als Aktivierungsfunktion $F_o$ kann für die meisten Anwendungen, insbesondere den hier betrachteten Fall der Ermittelung der Überlebensfunktion für Krebspatienten, die Identitätsfunktion $F_o(x) = x$ verwendet werden.

Die Antwortsignale $A_o$ der Ausgangs-Neuronen $N_o$ geben den jeweiligen Koeffizienten des zugehörigen Terms der Reihenentwicklung der gesuchten Überlebensfunktion an.

## 5.3. Die Überlebensfunktion

Wie vorstehend bereits angesprochen wurde, umfassen die Eingangsdaten Angaben über die Person des Patienten, sowie Angaben über das Krankheitsbild. Alle diese Daten werden zu einem Zeitpunkt t = O erfaßt, im Falle von Krebspatienten beispielsweise dem Zeitpunkt der Erstoperation. Im Anschluß an die Erstoperation werden die Patienten dann einer bestimmten Nachbehandlung unterzogen, die beispielsweise Chemo- oder/und Strahlen-Therapien beinhalten kann.

Die Überlebensfunktion S(t) gibt nun die Wahrscheinlichkeit an, daß bei einem betrachteten Patienten zum Zeitpunkt t ein bestimmtes Ereignis noch nicht eingetreten ist. Es kann sich bei diesem bestimmten Ereignis beispielsweise um eine erneute Krebserkrankung, im schlimmsten Fall aber auch um den Tod des Patienten handeln. In jedem Fall gilt für die Überlebensfunktion, daß S(0) = 1. Darüber hinaus wird üblicherweise $S(\infty) = 1$ angenommen.

Gemäß der üblichen Notation kann man auf Grundlage der Überlebensfunktion S(t) eine Ereignisdichte f(t) und eine Risikofunktion $\lambda(t)$ definieren:

$$f(t) = -dS/dt$$

$$\lambda(t) = f(t)/S(t)$$

woraus folgt:

$$\lambda(t) = -(d/dt)[\ln S(t)]$$

Kennt man also den Verlauf der Risikofunktion $\lambda(t)$, so kann man den Verlauf der Überlebensfunktion S(t) durch Integration rekonstruieren.

Es ist die Aufgabe des neuronalen Netzes, den Verlauf der Risikofunktion $\lambda(t)$ nach Art einer Reihenentwicklung zu modellieren:

$$\lambda(t) = \lambda_0 \cdot \exp[\sum_o B_o(t) \cdot A_o]$$

Dabei bezeichnen die Parameter $A_o$ gemäß vorstehender Notation die Antwortsignale der Neuronen $N_o$ der Ausgangsschicht des neuronalen Netzes. $\lambda_o$ ist im Rahmen der vorliegenden Erfindung ein von t unabhängiger Parameter, der als Normierungsfaktor eingesetzt wird. Mit $B_o(t)$ ist ein Satz von Funktionen bezeichnet, die als Basisfunktionen der Reihenentwicklung eine gute Annäherung an den tatsächlichen Verlauf der Risikofunktion ermöglichen. Als Funktionensatz $B_o(t)$ können beispielsweise die fraktalen Polynome oder auch Funktionen wie $t^P$ (p nicht notwendigerweise ganzzahlig) eingesetzt werden. Im Rahmen der vorliegenden Erfindung wurden $B_{o1}(t) = 1$; $B_{o2}(t) = const \cdot t^{1/2}$, ... verwendet.

### 5.4. Training des neuronalen Netzes - Vorbereitungen

5.4.1. Die Optimierungsfunktion

Die Trainingsdatenmenge umfaßt die Datensätze einer Mehrzahl von Patienten, für die nicht nur die Angaben über die Person und die Angaben über das Krankheitsbild bekannt sind, sondern auch Angaben über die Art der Nachbehandlung und der weitere Krankheitsverlauf. Aus den gesammelten Daten über den weiteren Krankheitsverlauf wird nach den folgenden Regeln eine "tatsächliche Überlebensfunktion" zusammengesetzt: Ist für einen bestimmten Patienten zu einem Zeitpunkt t das vorbestimmte Ereignis, beispielsweise eine erneute Erkrankung oder der Tod des Patienten bereits eingetreten, so wird sein Beitrag $\delta$ zur "tatsächlichen Überlebensfunktion" vor dem Zeitpunkt t zu $\delta = 0$ und ab dem Zeitpunkt t zu $\delta = 1$ gesetzt. Patienten, bei denen bis zum Zeitpunkt der Erhebung der Trainingsdatenmenge das vorbestimmte Ereignis noch nicht eingetreten ist ("zensierte" Daten), tragen zu jedem Zeitpunkt zur "tatsächlichen Überlebensfunktion" nur $\delta = 0$ bei. Während der Trainingsphase werden nun die Gewichte $w_{xy}$ der Synapsen und die sonstigen vorstehend unter Punkt 5.2. genannten Optimierungsparameter derart eingestellt, daß die vom neuronalen Netz gelieferte Überlebensfunktion möglichst gut der "tatsächlichen Überlebensfunktion" entspricht.

Dies kann beispielsweise dadurch erzielt werden, daß man eine für diesen Zweck geeignete Optimierungsfunktion O definiert und ein lokales, im günstigsten Fall sogar das globale Minimum dieser Optimierungsfunktion in dem von den Optimierungsparametern aufgespannten Raum sucht. Es ist im Stand der Technik bereits bekannt, zur Definition der Optimierungsfunktion O von einer sogenannten Likelihood-Funktion L auszugehen:

$$O = -\ln L$$

Erfindungsgemäß wird als Darstellung der Likelihood-Funktion

$$L = \prod_j [f_j(t)]^\delta \cdot [S_j(t)]^{1-\delta}$$

gewählt, wobei $f_j(t)$ und $S_j(t)$ gemäß der unter Punkt 5.3. eingeführten Notation die Ereignisdichte und die Überlebensfunktion für den Patienten j der Trainungsmenge bezeichnen. Diese Likelihood-Funktion hat den Vorteil, daß der Rechenaufwand nur etwa proportional zur Anzahl der in der Trainingsdatenmenge erfaßten Patienten ansteigt.

Eine weitere Möglichkeit zur Darstellung der Likelihood-Funktion ist:

$$L = \prod_j \left\langle \exp[\textstyle\sum_o B_o(t) \cdot A_{oj}] \ / \ \textstyle\sum_l \exp[\textstyle\sum_o B_o(t) \cdot A_{ol}] \right\rangle$$

wobei das Produkt über alle Patienten j gebildet wird, bei denen das vorbestimmte Ereignis zum Zeitpunkt t bereits aufgetreten ist, und wobei die erste Summe im Nenner des Quotienten über alle Patienten I gebildet wird, bei denen das vorbestimmte Ereignis zum Zeitpunkt t noch nicht aufgetreten ist.

Der mit dieser Darstellung verbundene Rechenaufwand steigt allerdings etwa proportional zum Quadrat der Anzahl der in der Trainingsdatenmenge erfaßten Patienten an.

5.4.2. Die Initialisierung

Zur Initialisierung der Netzoptimierungs-Parameter, beispielsweise der Gewichte der die Neuronen verbindenden Synapsen, können diesen Parametern, wie dies im Stand der Technik an sich bekannt ist, stochastisch kleine Werte zugewiesen werden, die gewissen Normierungsregeln gehorchen. Dabei ist es zusätzlich möglich, in die Normierung Erkenntnisse einfließen zu lassen, die vorab in Testläufen an neuronalen Netzen stark vereinfachter Struktur gewonnen wurden.

### 5.5. Training des neuronalen Netzes - Simplex-Methode

Die Suche nach einem lokoalen bzw. dem globalen Minimum der Optimierungsfunktion wird wie üblich in mehreren Schritten bzw. Zyklen durchgeführt. Erfindungsgemäß wird jedoch zu dieser Suche erstmals die von Nelder und Mead (siehe Punkt 6. "Literaturverzeichnis") vorgeschlagene Simplex-Methode bei einem neuronalen Netz eingesetzt. Unter einem Simplex wird in einem n-dimensionalen Raum ein (n + 1)-dimensionales Gebilde verstanden, das den aktuellen Stützpunkt in dem n-dimensionalen Raum umgibt, also in einem 2-dimensionalen Raum ein Dreieck, in einem 3-dimensionalen Raum eine Dreieckspyramide und so weiter. In welchen Richtungen und mit welchen Abständen vom aktuellen Stützpunkt die (n + 1) Eckpunkte angeordnet werden, wird dabei auf Grundlage der Eigenschaften der Optimierungsfunktion an den Eckpunkten des vorhergehenden Zyklus bestimmt.

Dieses Verfahren führt zu einer streng monoton abfallenden Folge von Stützpunkten. Es kann fortgesetzt werden, bis entweder (innerhalb vorgegebener Genauigkeitsgrenzen) ein lokales oder globales Minimum aufgefunden oder ein weiteres Abbruchkriterium erfüllt worden ist. Im Zusammenhang mit diesem weiteren Abbruchkriterium kommt nun die vorstehend bereits angesprochene Validierungs-Datenmenge ins Spiel:

Der vorstehend angesprochene monotone Abfall der Stützpunkte kann zum einen von tatsächlich objektivierbaren Eigenschaften der für die TrainingsDatenmenge bestimmten Optimerungsfunktion herrühren. Zum anderen ist es jedoch auch möglich, daß der abfallende Verlauf im Bereich einer von stochastischen Fluktuationen hervorgerufenen Senke der Optimierungsfunktion erfolgt. Der letztgenannte Effekt täuscht aber einen Lernerfolg nur vor. Daher werden erfindungsgemäß auch die Eigenschaften der anhand der Validierungs-Datenmenge bestimmten Optimierungsfunktion an den gleichen Stützpunkten untersucht. Stellt man dabei fest, daß auch die Stützpunkte der "Validierungsdaten-Optimierungsfunktion" monoton abfallen, so kann man davon ausgehen, daß man sich noch in einer "echten" Lernphase des neuronalen Netzes befindet. Stagniert die Folge der Stützpunkte der "Validierungsdaten-Optimierungsfunktion" hingegen oder steigt sie sogar wieder an, so muß davon ausgegangen werden, daß man sich bezüglich der "Trainingsdaten-Optimierungsfunktion" in einer von stochastischen Fluktuationen hervorgerufenen Senke befindet, die einen Lernfortschritt nur vorgaukelt. Die zyklische Durchführung der Simplex-Methode kann daher unterbrochen werden.

Der Vorteil der Simplex-Methode ist hauptsächlich darin zu sehen, daß sie allein anhand der Optimierungsfunktion durchgeführt werden kann und zudem die Schrittweite und Schrittrichtung automatisch festgelegt werden.

### 5.6. Training des neuronalen Netzes - Strukturvereinfachung ("Pruning")

Nachdem die Suche nach einem lokalen bzw. dem globalen Minimum abgeschlossen ist, wird gemäß einem nächsten Trainingsschritt untersucht, ob sich die Struktur des neuronalen Netzes aufgrund der bislang gewonnenen Erkenntnisse nicht vereinfachen läßt. Bei diesem "Pruning" geht es also um die Untersuchung der Frage, welche der Synapsen so wenig Einfluß auf die Gesamtfunktion des neuronalen Netzes haben, daß auf sie verzichtet werden kann. Dies kann im einfachsten Fall beispielsweise dadurch erfolgen, daß das ihnen zugewiesene Gewicht ein für allemal auf Null gesetzt wird. Grundsätzlich ist es jedoch ebenso denkbar, das Gewicht der betreffenden Synapse auf einen festen Wert "einzufrieren". Vorteilhafterweise sollten sich Simplexoptimierungs-Schritte und Strukturvereinfachungs-Schritte in einem iterativen Verfahren abwechseln. Selbstverständlich wäre es wünschenswert, das neuronale Netz bereits nach dem Ausschluß einer einzigen Synapse einer erneuten Simplexoptimierung zu unterziehen. Dies ist jedoch im Hinblick auf die für das Training insgesamt erforderliche Zerit nicht vertretbar. In der Praxis hat es sich als günstiger Kompromiß erwiesen, während eines Strukturvereinfachungs-Schritts allenfalls 10% der zu Beginn dieses Schritts noch vorhandenen Synapsen zu entfernen.

Zur Strukturvereinfachung kommen erfindungsgemäß die beiden nachfolgend unter den Punkten 5.6.1. und 5.6.2. beschriebenen Verfahren zum Einsatz.

#### 5.6.1. Likelihood-Methode

Im Rahmen dieser Methode wird als Referenzwert zunächst der Wert der Likelihood-Funktion auf Grundlage der gesamten Struktur des neuronalen Netzes in seinem gegenwärtigen Trainingszustand berechnet, d.h. unter Verwendung der aktuellen Werte der Gewichte aller Synapsen. Anschließend wird der Einfluß einer vorgegebenen Synapse unterdrückt, d.h. der Wert des Gewichts dieser Synapse wird auf Null gesetzt. Sodann wird der Wert der Likelihood-Funktion für die so vereinfachte Netzstruktur berechnet, und das Verhältnis dieses Werts zum Referenzwert gebildet.

Hat man dieses Likelihood-Verhältnis für alle Synapsen berechnet, so beginnt man bei der Durchführung der nachfolgend beschriebenen Schritte bei derjenigen Synapse, für die der Wert des Likelihood-Verhältnisses am nächsten bei Eins liegt:

Nimmt man an, daß die Netzstruktur bereits um (x-1) Synapsen vereinfacht worden ist und nunmehr die Bedeutung der x. Synapse untersucht wird, so vergleicht man die folgenden drei Netzstruktur-Varianten: Erstens Gesamtstruktur des neuronalen Netzes in seinem gegenwärtigen Trainingszustand mit allen vor diesem Strukturvereinfachungs-Schritt noch vorhandenen Synapsen, zweitens Netzstruktur unter Ausschluß der (x-1) in diesem Strukturvereinfachungs-Schritt bereits unterdrückten Synapsen, und drittens Netzstruktur unter Ausschluß nunmehr auch der x. Synapse. Anschließend vergleicht man die dritte Strukturvariante mittels eines Signifikanz-Tests zum einen mit der ersten Strukturvariante (volle Struktur) und zum anderen mit der zweiten Strukturvariante ((x-1) Synapsen unterdrückt). Wenn auch nur einer der beiden Tests eine zu starke Abweichung der dritten Strukturvariante ergibt, so wird die entsprechende Synapse zumindest für den nächstfolgenden Simplexoptimierungs-Schritt beibehalten.

Als Signifikanz-Test kann beispielsweise der an sich bekannte CHI-QUADRAT-Test eingesetzt werden. Alternativ könnte dieser Signifikanz-Test auch mittels der ebenfalls an sich bekannten BOOT-STRAPPING-Methode durchgeführt werden. Die Verwendung des CHI-QUADRAT-Tests ist besonders dann günstig, wenn man die Reaktion des neuronalen Netzes auf Grundlage einer Likelihood-Funktion vermittelt. Die BOOT-STRAPPING-Methode eignet sich auch bei anderen Arten von Funktionen zur Darstellung der Reaktion des neuronalen Netzes.

### 5.6.2. Korrelations-Methode

Der Ausschluß bzw. die Unterdrückung von Synapsen nach der Korrelations-Methode beruht auf der Überlegung, daß es möglich sein könnte, daß zwei in ein und derselben Schicht angeordnete Neuronen auf ein Neuron einer darunter angeordneten Schicht qualitativ den gleichen Einfluß ausüben. In diesem Fall sollte sich die Reaktion des neuronalen Netzes, genauer gesagt das Antwortsignal dieses letztgenannten Neurons, im wesentlichen nicht ändern, wenn man dieses Neuron lediglich von einem der beiden darüber angeordneten Neuronen stimulieren läßt und den Einfluß des zweiten Neurons durch eine Stärkung der verbleibenden Synapse berücksichtigt. Auf die von dem zweiten Neuron zu dem betrachteten Neuron führende Synapse könnte dann verzichtet werden.

**a. Eingangs-Neuronen und Ausgangs-Neuronen verbindende Synapsen**

Gemäß Punkt 5.2.4. hat der Beitrag des Antwortsignals zweier Eingangs-Neuronen zum Stimulationssignal eines Ausgangs-Neurons die Form:

$$S_o = w_{1o} \cdot (A_1 - c_1) + w_{2o} \cdot (A_2 - c_2)$$

Unterstellt man nun, daß die beiden Antwortsignale $A_1$ und $A_2$ gemäß

$$A_2 = m \cdot A_1 + n$$

zumindest näherungsweise miteinander korreliert sind und daß das Gewicht $w_{1o}$ größer ist als das Gewicht $w_{2o}$, so gilt für das Stimulationssignal $S_o$:

$$S_o = (w_{1o} + w_{2o} \cdot m) \cdot A_1 + (n \cdot w_{2o} - w_{1o} \cdot c_1 - w_{2o} \cdot c_2)$$

$$= w^{\cdot}_{1o} \cdot (A_1 - c^{\cdot}_1)$$

mit

$$w^{\cdot}_{1o} = w_{1o} + w_{2o} \cdot m$$

und

$$c^{\bullet}_{1} \quad = -[(n \cdot w_{2o} - w_{1o} \cdot c_1 - w_{2o} \cdot c_2)]/(w_{1o} + w_{2o} \cdot m)$$

Ist $w^*_{1o}$ nicht klein, so kann man das Verhalten des neuronalen Netzes unter den folgenden Annahmen testen:

1. Ersetze das Gewicht $w_{1o}$ durch $w^*_{1o}$;
2. Ersetze den Parameter $c_1$ durch $c^*_1$; und
3. Unterdrücke die Synapse vom Eingangs-Neuron $N_2$ zum Ausgangs-Neuron $N_o$.

Verläuft dieser Test, der beispielsweise wieder als CHI-QUADRAT-Test durchgeführt werden kann, positiv, so kann auf die Synapse vom Eingangs-Neuron $N_2$ zum Ausgangs-Neuron $N_o$ verzichtet werden.

**b. Eingangs-Neuronen und Zwischen-Neuronen verbindende Synapsen**

In analoger Weise kann man auch den Beitrag des Antwortsignals zweier Eingangs-Neuronen zum Stimulationssignal eines Zwischen-Neurons betrachten, wobei es sich, wie nachstehend sofort klar werden wird, empfiehlt, das Stimulationssignal des Zwischenneurons einschließlich dessen "Vorspannung" zu betrachten:

$$S_h - b_h = w_{1h} \cdot A_1 + w_{2h} \cdot A_2$$

Unterstellt man nun wiederum, daß die beiden Antwortsignale $A_1$ und $A_2$ gemäß

$$A_2 = m \cdot A_1 + n$$

zumindest näherungsweise miteinander korreliert sind und daß das Gewicht $w_{1h}$ größer ist als das Gewicht $w_{2h}$, so gilt für das Stimulationssignal $S_h$:

$$S_h - b_h = (w_{1h} + w_{2h} \cdot m) \cdot A_1 + n \cdot w_{2h}$$

oder

$$S_h - b^{\bullet}_h = w^{\bullet}_{1h} \cdot A_1$$

mit

$$w^{\bullet}_{1h} = w_{1h} + w_{2h} \cdot m$$

und

$$b^{\bullet}_h = b_h + n \cdot w_{2h}$$

Ist $w^*_{1h}$ nicht klein, so kann man das Verhalten des neuronalen Netzes unter den folgenden Annahmen testen:

1. Ersetze das Gewicht $w_{1h}$ durch $w^*_{1h}$;
2. Ersetze die Vorspannung $b_h$ durch $b^*_h$; und
3. Unterdrücke die Synapse vom Eingangs-Neuron $N_2$ zum Zwischen-Neuron $N_h$.

Verläuft dieser Test, der beispielsweise wieder als CHI-QUADRAT-Test durchgeführt werden kann, positiv, so kann auf die Synapse vom Eingangsneuron $N_2$ zum Zwischenneuron $N_h$ verzichtet werden.

**c. Zwischen-Neuronen und Ausgangs-Neuronen verbindende Synapsen**

In analoger Weise können auch von Zwischen-Neuronen zu Ausgangs-Neuronen führende Synapsen behandelt werden. Hinsichtlich der Vorspannungen $b_o$ ist jedoch möglicherweise die unter Punkt 5.2.4. angesprochene weitere Randbedingung zu beachten.

### 5.6.3. Überprüfung der Topologie

Das vorstehend beschriebene Ausdünnen der Struktur des neuronalen Netzes kann zur Folge haben, daß einzelne Neuronen mit keinem einzigen anderen Neuron mehr in Verbindung stehen. Dies ist beispielsweise der Fall, wenn ein Eingangs-Neuron mit keinem Zwischen-Neuron und auch keinem Ausgangs-Neuron in Verbindung steht, oder wenn ein Ausgangs-Neuron mit keinem Zwischen-Neuron und auch keinem Eingangs-Neuron in Verbindung steht. Es ist daher nur folgerichtig, wenn diese Neuronen, die keinen Einfluß auf die Funktion des neuronalen Netzes mehr haben, vollständig deaktiviert werden.
Einen Spezialfall bilden Zwischen-Neuronen, die zwar noch mit Neuronen der Eingangsschicht in Verbindung stehen, nicht jedoch mit Neuronen der Ausgangsschicht. Diese Zwischen-Neuronen können auf die Funktion des neuronalen Netzes keinerlei Einfluß mehr ausüben. Daher können auch die von der Eingangsschicht zu diesen Zwischen-Neuronen führenden Synapsen unterdrückt werden, d.h. die Gewichte dieser Synapsen auf Null gesetzt werden.
Es kann aber auch der umgekehrte Fall auftreten, nämlich daß ein Zwischen-Neuron zwar noch mit der Ausgangsschicht verbunden ist, jedoch keinerlei Verbindung mehr mit der Eingangsschicht hat. Diese Zwischen-Neuronen können allenfalls ein von ihrer "Vorspannung" abhängendes Antwortsignal an die Ausgangs-Neuronen abgeben. Ein derartiges Signal hat aber keinerlei Informationsgehalt, der für die Funktion des neuronalen Netzes von Bedeutung wäre. Somit können auch die restlichen Synapsen dieser Zwischen-Neuronen unterdrückt werden.

### 5.7. Generalisierung

Nach Abschluß der Trainingsphase ist es erforderlich, die Leistungsfähigkeit des trainierten neuronalen Netzes zu überprüfen, um ein Maß dafür zu bekommen, welche Aussagekraft die von diesem neuronalen Netz gelieferten Überlebensfunktionen tatsächlich haben. Hierzu wird die vorstehend bereits angesprochene Generalisierungs-Datenmenge eingesetzt, die auf das Training des neuronalen Netzes keinerlei Einfluß genommen hat und somit eine objektive Aussage ermöglicht.

### 5.8. Abschließende Bemerkungen

Abschließend sei erwähnt, daß neben den vorstehend explizit angesprochenen tumor-spezifischen Faktoren upA und PAI-1, die Aussagen über die Invasion zulassen, auch weitere derartige Faktoren berücksichtigt werden können. Es sind dies unter anderem Faktoren für die Proliferation, beispielsweise die S-Phase und Ki-67, sowie für weitere das Tumorwachstum beeinflussende Prozesse.

### 6. LITERATURVERZEICHNIS

Zum Stand der Technik wird auf die folgenden Druckschriften verwiesen:

1. Barnhill S, Zhang Z, US 5,769,074, 'Computer assisted methods for diagnosing diseases'.

2. Bellotti M, et al. (1997), 'Neural networks as a prognostic tool for patients with non-small cell carcinoma of the lung', Mod Pathol. Dec, 10(12), 1221.

3. Biganzoli, E. Boracchi, P. Mariani, L., Marubini, E. (1998), 'Feed Forward Neural Networks for the Analysis of Censored Survival Data: A Partial Logistic Regression Approach', Statistics in Medicine, 17, 1169.

4. Bostwick DG (1998), 'Practical clinical application of predictive factors in prostate cancer: A review with an emphasis on quantitative methods in tissue specimens', Anal Quant Cytol Histol. Oct, 20(5), 323-42. Review.

5. Bottaci L, et al. (1997), 'Artificial neural networks applied to outcome prediction for colorectal cancer patients in separate institutions', Lancet. Aug 16, 350, 469.

6. Bryce TJ, et al(1998), 'Artificial neural network model of survival in patients treated with irradiation with and without concurrent chemotherapy for advanced carcinoma of the head and neck', Int J Radiat Oncol Biol Phys. May 1, 41 (2), 339.

7. Burke HB, et al. (1997), 'Artificial neural networks improve the accuracy of cancer survival prediction', Cancer. Feb 15, 79(4), 857.

8. Cox, D. (1972) 'Regression Models And Life Tables', J R Stat Soc [B] 34, 187.

9. Comanor L, Minor J, US 5,860,917, 'Method and apparatus for predicting therapeutic outcomes'.

10. De Laurentiis, M. & Ravdin, P. (1994), 'Survival Analysis of Censored Data, Neural Network Analysis Detection of Complex Interactions Between Variables', Breast Cancer Res Tr 32, 113.

11. Ebell, M. (1993), 'Artificial Neural Network Model for Predicting Failure to Survive Following In-Hospital Cardiopulmonary Resuscitation', The Journal of Family Practice, 36, 297.

12. Faraggi, D. & Simon, R. (1995), 'A Neural Network Model for Survival Data', Statistics in Medicine, 14, 73.

13. Faraggi, D. Simon, R., Yaskil, E., Kramar A., (1997), 'Bayesian Neural Network Models for Censored Data', Biometrical Journal, 39, 519.

14. Fogel, D., Wasson, E., & Boughton, E. (1995), 'Evolving Neural Networks for Detecting Breast Cancer', Cancer Letters, 96, 49.

15. Gray, R. (1992) 'Flexible Methods for Analyzing Survival Data Using Splines, with Applications to Breast Cancer Prognosis', J. American Statistical Association, 87, 942.

16. Hamamoto I, et al. (1995), 'Prediction of the early prognosis of the hepatectomized patient with hepatocellular carcinoma with a neural network', Comput Biol Med. Jan, 25(1), 49.

17. Hastie, T., Sleeper, L. & Tibshirani, R. (1992) 'Flexible Covariate Effects in the Proportional Hazards Model', Breast Cancer Res Tr 22, 241.

18. Hilsenbeck, S., & Clark, G. (1996), 'Practical p-Value Adjustment for Optimally Selected Cutpoints', Statistics in Medicine, 15, 103.

19. Hilsenbeck, S., Clark, G., & McGuire, W. (1992), 'Why Do So Many Prognostic Factors Fail to Pan Out?', Breast Cancer Res Tr 22, 197.

20. Jefferson MF, et al. (1997), 'Comparison of a genetic algorithm neural network with logistic regression for predicting outcome after surgery for patients with nonsmall cell lung carcinoma', Cancer. Apr 1, 79(7), 1338.

21. Kalbfleisch, J. & Prentice, R. (1980), The Statistical Analysis of Failure Time Data, Wiley.

22. Kappen HJ, et al. (1993), 'Advanced ovarian cancer: Neural network analysis to predict treatment outcome', Ann Oncol., 4 Suppl 4, 31.

23. Kharchenko EP. (1996), '[The use of a neural network model for cancer prognosis on the basis of immunological indices]', Biull Eksp Biol Med. Aug, 122(8), 206. Russian.

24. Knorr, K., Hilsenbeck, S., Wenger, C. et al., (1992) 'Making the Most of Your Prognostic Factors, Presenting a More Accurate Survival Model for Breast Cancer Patients', Breast Cancer Res Tr 22, 251.

25. Koutsoukos AD, et al. (1994), 'Discrimination techniques applied to the NCI in vitro anti-tumour drug screen: predicting biochemical mechanism of action', Stat Med. Mar 15-Apr 15, 13(5-7), 719.

26. Le Cun, Y., Denker, J., Solla, S. (1990), 'Optimal Brain Damage', in Advances in Neural Information Processing Systems 2, (Hrsg. D. Touretzky), San Mateo

27. Liestol, K., Anderson, P. & Anderson, U. (1994), 'Survival Analysis and Neural Nets', Statistics in Medicine, 13, 1 189.

28. Mariani L, et al. (1997), 'Prognostic factors for metachronous contralateral breast cancer, a comparison of the linear Cox regression model and its artificial neural network extension', Breast Cancer Res Treat. Jun, 44(2), 167.

29. Marsh JW, et al. (1998), 'Liver transplantation in the treatment of hepatocellular carcinoma', J Hepatobiliary Pancreat Surg., 5(1), 24.

30. Marsh JW, et al. (1997), 'The prediction of risk of recurrence and time to recurrence of hepatocellular carcinoma after orthotopic liver transplantation, a pilot study. Hepatology. Aug, 26(2), 444.

31. McGuire WL, et al. (1992), 'Treatment decisions in axillary node-negative breast cancer patients', J Natl Cancer Inst Monogr., (11), 173.

32. McGuire, W., Tandon, A., Allred, D. Chamness, G. & Clark, G. (1990), 'How To Use Prognostic Factors in Axillary Node-Negative Breast Cancer Patients ', J. Natl Canc Inst 82, 1006.

33. Naguib RN, et al. (1998), 'Neural network analysis of combined conventional and experimental prognostic markers in prostate cancer: a pilot study' Br J Cancer. Jul, 78(2), 246.

34. Naguib RN, et al. (1997), 'Prediction of nodal metastasis and prognosis in breast cancer: a neural model', Anticancer Res. Jul-Aug, 17(4A), 2735.

35. Nelder, J.A., & Mead, R. (1965), ' A simplex method for function minimization', Computer Journal 7, 308.

36. Nowlan, S. J. (1991), Soft Competitive Adaption, Neural Network Learning Algorithms based on Fitting Statistical Mixtures, PhD Thesis, School of Comp. Sc., Carnegie Mellon, Pittsburgh.

37. Press, W., Teukolsky, S., Flannery, B. & Vetterling, W. (1992), Numerical Recipies, The Art of Scientific Computing, Cambridge.

38. Ravdin, P. & Clark, G. (1992) 'A Practical Application of Neural Network Analysis for Predicting Outcome of Individual Breast Cancer Patients' , Breast Cancer Res Tr 22, 285.

39. Ravdin, P. Clark, G. Hilsenbeck, S. , et al. (1992), 'A Demonstration that Breast Cancer Recurrence can be Predicted by Neural Network Analysis', Breast Cancer Res Tr 21,47.

40. Ravdin, P. McGuire, W., Clark, G., US 5,862,304, 'Method for predicting the future occurrence of clinically occult or non-existent medical conditions'.

41. Rehkugler, H. & Zimmerman, H. (1994) Neuronale Netze in der Ökonomie, Vahlen Verlag.

42. Ripley, B. (1993) 'Statistical Aspects of Neural Networks', in Networks and Chaos - Statistical and Probabilistic Aspects, Hrsg. Barndorff-Nielsen, O. Jensen, J. & Kendall, W., Chapman & Hill.

43. Ritter, H., Martinetz, T. & Schulten, K. (1990), Neuronale Netze, Eine Einführung in die Neuroinformatik Selbstorganisierender Netzwerke, Addison-Wesley.

44. Royston, P. & Altman. D. (1994), 'Regression using fractional polynomials of continuous covariates, parsimonious parametric modelling', App. Stat. 43, 429.

45. Schumacher, M., Rossner, R. Vach, W., (1996), 'Neural Networks and logistic regression', Computational Statistics & Data Analysis, 21, 661 (Part I).

46. Snow PB, et al. (1994), 'Artificial neural networks in the diagnosis and prognosis of prostate cancer, a pilot study. J Urol. Nov, 152 (5 Pt 2), 1923.

47. Speight PM, et al. (1995), 'The use of artificial intelligence to identify people at risk of oral cancer and precancer', Br Dent J. Nov 25, 179(10), 382.

48. Tibshirani, R. (1996), 'A comparison of some error estimates for neural network procedures', Neural Computation 8, 152.

49. Tracey KJ, et al. (1992), 'Tumor necrosis factor in metabolism of disease, hormonal actions versus local tissue effects', Nouv Rev Fr Hematol. , 34 Suppl, S37.

50. Tu, J. (1996) 'Advantages and Disadvantages of Using Artificial Neural Networks versus Logistic Regression for Predicting Medical Outcomes', J. Clin. Epidemiol. 49, 1225.

51. Warner, B. & Misra, M, (1996), 'Understanding Neural networks as statistical tools,' The American Statistician 50, 284.

52. Willoughby TR, et al. (1996), 'Evaluation and scoring of radiotherapy treatment plans using an artificial neural network', Int J Radiat Oncol Biol Phys. Mar 1, 34(4), 923.

**Patentansprüche**

1. Verfahren zum Trainieren eines neuronalen Netzes zur Ermittelung einer Risikofunktion $\lambda$ (t) für Patienten im Anschluss an eine Ersterkrankung mit einer vorbestimmten Krankheit auf Grundlage vorgegebener Trainings-Datensätze, welche objektivierbare und messtechnisch erfasste Daten zum Krankheitsbild der Patienten beinhalten, wobei das neuronale Netz umfasst:

   - eine Eingangsschicht mit einer Mehrzahl von Eingangs-Neuronen,
   - wenigstens eine Zwischenschicht mit einer Mehrzahl von Zwischen-Neuronen,
   - eine Ausgangsschicht mit einer Mehrzahl von Ausgangs-Neuronen, und
   - eine Vielzahl von Synapsen, die jeweils zwei Neuronen unterschiedlicher Schichten miteinander verbinden,

   wobei
   das Training des neuronalen Netzes eine Strukturvereinfachungs-Prozedur zum Auffinden und Eliminieren von Synapsen, welche auf den Verlauf der
   Risikofunktion keinen wesentlichen Einfluss ausüben, umfasst, **dadurch gekennzeichnet, dass**

   a1) man zwei mit ein und demselben Empfangs-Neuron verbundene Sende-Neuronen auswählt,
   a2) man die Synapse eines der beiden Sende-Neuronen zu dem Empfangs-Neuron unterbricht und dafür das Gewicht der Synapse des jeweils anderen Sende-Neurons zu dem Empfangs-Neuron wie folgt anpasst:

   $w_{1o}{}^* = w_{1o} + w_{2o}$ m, wobei $w_{1o}{}^*$ das angepasste Gewicht, und $w_{1o}$ bzw. $w_{20}$ das Gewicht der Synapse von dem einen der beiden bzw. von dem anderen der beiden Sende-Neuronen zu dem Empfangs-Neuron mit $w_{10} > w_{2o}$ ist, und wobei sich m aus der Korrelation der Antwortsignale des einen und des anderen der beiden Sende-Neuronen $A_1$ und $A_2$ gemäß $A_2 = m A_1 + n$ ergibt;
   a3) man das Antwortsignal des Empfangs-Neurons für das gemäß Schritt a2) veränderte neuronale Netz mit dem Antwortsignal des Empfangs-Neurons für das unveränderte neuronale Netz vergleicht, und
   a4) man dann, wenn die Abweichung der in Schritt a3) verglichenen Antwortsignale ein vorbestimmtes Maß nicht überschreitet, entscheidet, die in Schritt a2) vorgenommene Änderung beizubehalten; und
   die Risikofunktion $\lambda$(t) in Form einer Reihenentwicklung nach Antwortsignalen $A_o$ des trainierten neuronalen

Netzes der Ausgangs-Neuronen und mit Basisfunktionen der Reihenentwicklung

$$B_o(t): \quad \lambda(t) = \exp\left(\sum_o B_o(t) \cdot A_o\right) \text{ bestimmt wird.}$$

**2.** Verfahren nach Anspruch 1, in dem die beiden Sende-Neuronen in ein und derselben Schicht angeordnet sind.

**3.** Verfahren nach Anspruch 1 oder 2, in dem man in Schritt a2) ferner den Wert der Vorspannung des Empfangs-Neurons, wenn dieses ein Zwischen-Neuron ist und das Sende-Neuron eine Eingangs-Neuron ist, anpasst gemäß:

$b_h^* = b_h + n\, w_{2h}$, wobei gilt $S_h - b_h = w_{1h} A_1 + w_{2h} A_2$ mit der angepassten Vorspannung $b_h^*$, der unangepassten Vorspannung $b_h$ und dem Stimulationssignal für das Zwischen-Neuron $S_h$.

**4.** Verfahren nach einem der Ansprüche 1 bis 3, in dem man zur Darstellung der Reaktion des neuronalen Netzes den Wert einer Likelihood-Funktion für das neuronale Netz ermittelt.

**5.** Verfahren nach einem der Ansprüche 1 bis 4, in dem man den Vergleich von Strukturvarianten des neuronalen Netzes mittels eines Signifikanz-Tests durchführt.

**6.** Verfahren nach Anspruch 5, in dem man den Vergleich von Strukturvarianten des neuronalen Netzes mittels des an sich bekannten CHI-QUADRAT-Tests durchführt.

**7.** Verfahren nach Anspruch 5, in dem man den Vergleich von Strukturvarianten des neuronalen Netzes mittels der an sich bekannten BOOT-STRAPPING-Methode durchführt.

**8.** Verfahren nach einem der Ansprüche 1 bis 6, in dem man zum Vergleich zweier Strukturvarianten des neuronalen Netzes das Verhältnis der Werte der Likelihood-Funktionen für diese beiden Strukturvarianten ermittelt.

**9.** Verfahren nach einem der Ansprüche 1 bis 8, in dem das Training des neuronaten Netzes eine Optimierungs-Prozedur umfasst, in welcher die Stärken der einzelnen Synapsen, d. h. die Stärken der Verbindungen zwischen den Neuronen, optimiert werden, und in dem zu dieser Optimierung die an sich bekannte Simplex-Methode eingesetzt wird.

**Claims**

**1.** A method for training a neural network in order to identify a risk function $\lambda(t)$ for patients following a first occurrence of a pre-specified disease on the basis of given training data records containing objectifiable data acquired by measurements relating to the medical condition of the patients, wherein the neural network includes

- an input layer having a plurality of input neurons,
- at least one hidden layer having a plurality of hidden neurons
- an output layer having a plurality of output neurons,
- a plurality of synapses, wherein each said synapse connects two neurons of different layers

wherein the training of the neural network comprises a procedure for simplification of structure for the identification and elimination of synapses whose influence on the form of risk function form is inferred to be insignificant **characterized by**

a1) selecting two sending neurons that are connected to the same receiving neuron a2) capping one of the two synapses of said sending neurons and adapting in its place the weight of the synapse of the respective other sending neuron to the receiving neuron as follows:

$$w_{1o}^* = w_{1o} + w_{2o}\, m,$$

where $w_{1o}^*$ is the adapted weight; $w_{1o}$ and $w_{2o}$, with $w_{1o} > w_{2o}$, denote the weights of the synapses connecting each of said two neurons to the receiving neuron, respectively; and where m is obtained from the correlation of the response signals of the corresponding sending neurons $A_1$ and $A_2$ by $A_2 = m\,A_1 + n$;

a3) comparing the response signal of the receiving neuron for the neural network modified according to a2) with the response signal of the receiving neuron for the unmodified neural network; and

a4) deciding to accept the modification of step a2) provided that said two response signals compared in step a3) do not differ by more than a pre-determined measure; and in that the risk function $\lambda(t)$ is determined in the form of a series expansion with output neuron response signals $A_o$ of the trained neural net and basis functions $B_o(t)$ of the series expansion:

$$\lambda(t) = \exp\left( \sum_o \left( B_o(t) A_o \right) \right)$$

2. The method of Claim 1, in which said two sending neurons are located in the same layer.

3. The method of Claim 1 or 2, wherein step a2) further includes adapting a value of the receiving neuron bias, if it is a hidden-layer neuron and the sending neuron is an input layer neuron, according to.

$$b_h{}^* = b_h + n\,w_{2h},$$

with

$$S_h - b_h = w_{1h}\,A_1 + w_{2h}\,A_2\,,$$

where $b_h{}^*$ denotes the adapted bias, $b_h$ denotes the pre-change bias, and $S_h$ denotes the stimulation signal of the hidden neuron.

4. The method of Claims 1 to 3, in which the value of a likelihood function is calculated to represent the reaction of the neural network.

5. The method of any one of Claims 1 to 4, in which comparison of structure variants of the neural network is carried out using a significance test.

6. The method of Claim 5, in which comparison of structure variants of the neural network is carried out using the known Chi-Squared test.

7. The method of Claim 5, in which comparison of structure variants of the neural network is carried out using the known Bootstrap method.

8. The method of any one of Claims 1 to 6, in which, for comparison of two structure variants of the neural network, the ratio of the values of the likelihood functions for said two structure variants is calculated.

9. The method of any one of Claims 1 to 8, in which neural network training comprises an optimization procedure, in which the strengths of the individual synapses - i.e., the weights of the neural connections - are optimized, whereby the known simplex method is utilized for said optimization.

**Revendications**

1. Procédé pour l'apprentissage d'un réseau neuronal pour déterminer une fonction de risque $\lambda(t)$ pour des patients après qu'ils ont été atteints pour la première fois d'une maladie prédéfinie, sur la base d'ensembles prédéfinis de données d'apprentissage qui contiennent des données, objectivables et relevées à l'aide d'une technique de mesure, portant sur le tableau clinique des patients, le réseau neuronal comprenant :

- une couche d'entrée avec plusieurs neurones d'entrée,
- au moins une couche intermédiaire avec plusieurs neurones intermédiaires,
- une couche de sortie avec plusieurs neurones de sortie, et
- un grand nombre de synapses qui relient chacune deux neurones de couches différentes,

étant précisé que l'apprentissage dans le réseau neuronal comprend une procédure de simplification de structure pour trouver et éliminer des synapses qui n'exercent pas d'influence notoire sur la courbe de la fonction de risque, **caractérisé**

a1) en ce qu'on sélectionne deux neurones émetteurs reliés à un même neurone récepteur,

a2) en ce qu'on interrompt la synapse de l'un des deux neurones émetteurs avec le neurone récepteur et on adapte pour cela le poids de la synapse de l'autre neurone émetteur avec le neurone récepteur de la façon suivante :

$w_{1o}{}^* = w_{1o} + w_{2o} \cdot m$, $w_{1o}{}^*$ désignant le poids adapté, et $w_{1o}$ et $w_{20}$ le poids de la synapse d'un neurone émetteur et de l'autre neurone émetteur avec le neurone récepteur, avec $w_{1o} > w_{2o}$, et m résultant de la corrélation des signaux de réponse d'un neurone émetteur et de l'autre neurone émetteur $A_1$ et $A_2$, selon $A_2 = m \cdot A_1 + n$ ;

a3) on compare le signal de réponse du neurone récepteur pour le réseau neurone modifié selon l'étape a2) au signal de réponse du neurone récepteur pour le réseau neuronal inchangé, et

a4) on décide, si l'écart des signaux de réponse comparés lors de l'étape a3) ne dépasse pas une valeur prédéfinie, de maintenir la modification apportée lors de l'étape a2) ; et

la fonction de risque $\lambda(t)$ est définie sous la forme d'un développement en série selon les signaux de réponse $A_o$ des neurones de sortie du réseau neuronal soumis à l'apprentissage, et avec des fonctions de base du développement en série

$$B_o(t) : \lambda(t) = \exp\left( \sum_o B_o(t) \cdot A_o \right) .$$

2. Procédé selon la revendication 1, selon lequel les deux neurones émetteurs sont disposés dans une même couche.

3. Procédé selon la revendication 1 ou 2, selon lequel on adapte par ailleurs, lors de l'étape a2), la valeur de la polarisation du neurone récepteur si celui-ci est un neurone intermédiaire et si le neurone émetteur est un neurone d'entrée, selon :

$b_h{}^* = b_h + n \cdot w_{2h}$, avec $S_h - b_h = w_{1h} \cdot A_1 + w_{2h} \cdot A_2$ avec la polarisation adaptée $b_h{}^*$, la polarisation non adaptée $b_h$ et le signal de stimulation pour le neurone intermédiaire $S_h$.

4. Procédé selon l'une des revendications 1 à 3, selon lequel pour représenter la réaction du réseau neuronal, on détermine la valeur d'une fonction de probabilité pour le réseau neuronal.

5. Procédé selon l'une des revendications 1 à 4, selon lequel on effectue la comparaison de variantes de structure du réseau neuronal à l'aide d'un test de signification.

6. Procédé selon la revendication 5, selon lequel on effectue la comparaison de variantes de structure du réseau neuronal à l'aide du test du khi carré connu en soi.

7. Procédé selon la revendication 5, selon lequel on effectue la comparaison de variantes de structure du réseau neuronal à l'aide de la méthode du boot-strapping connue en soi.

8. Procédé selon l'une des revendications 1 à 6, selon lequel pour comparer deux variantes de structure du réseau neuronal, on détermine le rapport des valeurs des fonctions de probabilité pour ces deux variantes de structure.

9. Procédé selon l'une des revendications 1 à 8, selon lequel l'apprentissage dans le réseau neuronal comprend une procédure d'optimisation dans laquelle les puissances des synapses individuelles, c'est-à-dire les puissances des connexions entre les neurones, sont optimisées, et selon lequel on utilise pour cette optimisation la méthode Simplex

connue en soi.

Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5734797 A
- US 5769074 A, Barnhill S, Zhang Z
- US 5860917 A, Comanor L, Minor J

- US 5862304 A, Ravdin, P. McGuire, W., Clark, G.

**In der Beschreibung aufgeführte Nicht-Patentliteratur**

- **Bellotti M et al.** Neural networks as a prognostic tool for patients with non-small cell carcinoma of the lung. *Mod Pathol.,* Dezember 1997, vol. 10 (12), 1221
- **Biganzoli, E. ; Boracchi, P. ; Mariani, L. ; Marubini, E.** Feed Forward Neural Networks for the Analysis of Censored Survival Data: A Partial Logistic Regression Approach. *Statistics in Medicine,* 1998, vol. 17, 1169
- **Bostwick DG.** Practical clinical application of predictive factors in prostate cancer: A review with an emphasis on quantitative methods in tissue specimens. *Anal Quant Cytol Histol.,* Oktober 1998, vol. 20 (5), 323-42
- **Bottaci L et al.** Artificial neural networks applied to outcome prediction for colorectal cancer patients in separate institutions. *Lancet.,* 16. August 1997, vol. 350, 469
- **Bryce TJ et al.** Artificial neural network model of survival in patients treated with irradiation with and without concurrent chemotherapy for advanced carcinoma of the head and neck. *Int J Radiat Oncol Biol Phys.,* 01. Mai 1998, vol. 41 (2), 339
- **Burke HB et al.** Artificial neural networks improve the accuracy of cancer survival prediction. *Cancer.,* 15. Februar 1997, vol. 79 (4), 857
- **Cox, D.** Regression Models And Life Tables. *J R Stat Soc [B],* 1972, vol. 34, 187
- **De Laurentiis, M. ; Ravdin, P.** Survival Analysis of Censored Data, Neural Network Analysis Detection of Complex Interactions Between Variables. *Breast Cancer Res Tr,* 1994, vol. 32, 113
- **Ebell, M.** Artificial Neural Network Model for Predicting Failure to Survive Following In-Hospital Cardiopulmonary Resuscitation. *The Journal of Family Practice,* 1993, vol. 36, 297
- **Faraggi, D. ; Simon, R.** A Neural Network Model for Survival Data. *Statistics in Medicine,* 1995, vol. 14, 73
- **Faraggi, D. ; Simon, R. ; Yaskil, E. ; Kramar A.** Bayesian Neural Network Models for Censored Data. *Biometrical Journal,* 1997, vol. 39, 519

- **Fogel, D. ; Wasson, E. ; Boughton, E.** Evolving Neural Networks for Detecting Breast Cancer. *Cancer Letters,* 1995, vol. 96, 49
- **Gray, R.** Flexible Methods for Analyzing Survival Data Using Splines, with Applications to Breast Cancer Prognosis. *J. American Statistical Association,* 1992, vol. 87, 942
- **Hamamoto I et al.** Prediction of the early prognosis of the hepatectomized patient with hepatocellular carcinoma with a neural network. *Comput Biol Med.,* Januar 1995, vol. 25 (1), 49
- **Hastie, T. ; Sleeper, L. ; Tibshirani, R.** Flexible Covariate Effects in the Proportional Hazards Model. *Breast Cancer Res Tr,* 1992, vol. 22, 241
- **Hilsenbeck, S. ; Clark, G.** Practical p-Value Adjustment for Optimally Selected Cutpoints. *Statistics in Medicine,* 1996, vol. 15, 103
- **Hilsenbeck, S. ; Clark, G. ; McGuire, W.** Why Do So Many Prognostic Factors Fail to Pan Out?. *Breast Cancer Res Tr,* 1992, vol. 22, 197
- **Jefferson MF et al.** Comparison of a genetic algorithm neural network with logistic regression for predicting outcome after surgery for patients with nonsmall cell lung carcinoma. *Cancer.,* 01. April 1997, vol. 79 (7), 1338
- **Kalbfleisch, J. ; Prentice, R.** The Statistical Analysis of Failure Time Data. Wiley, 1980
- **Kappen HJ et al.** Advanced ovarian cancer: Neural network analysis to predict treatment outcome. *Ann Oncol.,* 1993, vol. 4 (4), 31
- **Kharchenko EP.** The use of a neural network model for cancer prognosis on the basis of immunological indices. *Biull Eksp Biol Med.,* August 1996, vol. 122 (8), 206
- **Knorr, K. ; Hilsenbeck, S. ; Wenger, C. et al.** Making the Most of Your Prognostic Factors, Presenting a More Accurate Survival Model for Breast Cancer Patients. *Breast Cancer Res Tr,* 1992, vol. 22, 251

- **Koutsoukos AD et al.** Discrimination techniques applied to the NCI in vitro anti-tumour drug screen: predicting biochemical mechanism of action. *Stat Med.,* 15. Marz 1994, vol. 13 (5-7), 719
- Optimal Brain Damage. **Le Cun, Y. ; Denker, J. ; Solla, S.** Advances in Neural Information Processing Systems 2. 1990
- **Liestol, K. ; Anderson, P. ; Anderson, U.** Survival Analysis and Neural Nets. *Statistics in Medicine,* 1994, vol. 13 (1), 189
- **Mariani L et al.** Prognostic factors for metachronous contralateral breast cancer, a comparison of the linear Cox regression model and its artificial neural network extension. *Breast Cancer Res Treat.,* Juni 1997, vol. 44 (2), 167
- **Marsh JW et al.** Liver transplantation in the treatment of hepatocellular carcinoma. *J Hepatobiliary Pancreat Surg.,* 1998, vol. 5 (1), 24
- **Marsh JW et al.** The prediction of risk of recurrence and time to recurrence of hepatocellular carcinoma after orthotopic liver transplantation, a pilot study. *Hepatology,* August 1997, vol. 26 (2), 444
- **McGuire WL et al.** Treatment decisions in axillary node-negative breast cancer patients. *J Natl Cancer Inst Monogr.,* 1992, 173
- **McGuire, W. ; Tandon, A. ; Allred, D. ; Chamness, G. ; Clark, G.** How To Use Prognostic Factors in Axillary Node-Negative Breast Cancer Patients. *J. Natl Canc Inst,* 1990, vol. 82, 1006
- **Naguib RN et al.** Neural network analysis of combined conventional and experimental prognostic markers in prostate cancer: a pilot study. *Br J Cancer.,* Juli 1998, vol. 78 (2), 246
- **Naguib RN et al.** Prediction of nodal metastasis and prognosis in breast cancer: a neural model. *Anticancer Res.,* Juli 1997, vol. 17 (4A), 2735
- **Nelder, J.A. ; Mead, R.** A simplex method for function minimization. *Computer Journal,* 1965, vol. 7, 308
- Soft Competitive Adaption, Neural Network Learning Algorithms based on Fitting Statistical Mixtures. **Nowlan, S. J.** PhD Thesis. School of Comp. Sc, 1991
- **Press, W. ; Teukolsky, S. ; Flannery, B. ; Vetterling, W.** Numerical Recipies. The Art of Scientific Computing, 1992
- **Ravdin, P. ; Clark, G.** A Practical Application of Neural Network Analysis for Predicting Outcome of Individual Breast Cancer Patients. *Breast Cancer Res,* 1992, vol. Tr 22, 285
- **Ravdin, P. ; Clark, G. ; Hilsenbeck, S. et al.** A Demonstration that Breast Cancer Recurrence can be Predicted by Neural Network Analysis. *Breast Cancer Res,* 1992, vol. Tr 21, 47
- **Rehkugler, H. ; Zimmerman, H.** Neuronale Netze in der Ökonomie. Vahlen Verlag, 1994
- Statistical Aspects of Neural Networks. **Ripley, B.** Networks and Chaos - Statistical and Probabilistic Aspects. Chapman & Hill, 1993
- **Ritter, H. ; Martinetz, T. ; Schulten, K.** Neuronale Netze, Eine Einführung in die Neuroinformatik Selbstorganisierender Netzwerke. Addison-Wesley, 1990
- **Royston, P. ; Altman. D.** Regression using fractional polynomials of continuous covariates, parsimonious parametric modelling. *App. Stat.,* 1994, vol. 43, 429
- **Schumacher, M. ; Rossner, R. ; Vach, W.** Neural Networks and logistic regression. *Computational Statistics & Data Analysis,* 1996, vol. 21, 661
- **Snow PB et al.** Artificial neural networks in the diagnosis and prognosis of prostate cancer, a pilot study. *J Urol.,* November 1994, vol. 152, 1923
- **Speight PM et al.** The use of artificial intelligence to identify people at risk of oral cancer and precancer. *Br Dent J.,* 25. November 1995, vol. 179 (10), 382
- **Tibshirani, R.** A comparison of some error estimates for neural network procedures. *Neural Computation,* 1996, vol. 8, 152
- **Tracey KJ et al.** Tumor necrosis factor in metabolism of disease, hormonal actions versus local tissue effects. *Nouv Rev Fr Hematol.,* 1992, vol. 34, 37
- **Tu, J.** Advantages and Disadvantages of Using Artificial Neural Networks versus Logistic Regression for Predicting Medical Outcomes. *J. Clin. Epidemiol.,* 1996, vol. 49, 1225
- **Warner, B. ; Misra, M.** Understanding Neural networks as statistical tools. *The American Statistician,* 1996, vol. 50, 284
- **Willoughby TR et al.** Evaluation and scoring of radiotherapy treatment plans using an artificial neural network. *Int J Radiat Oncol Biol Phys.,* 01. Marz 1996, vol. 34 (4), 923